# EUROPEAN PATENT APPLICATION

(11) **EP 3 190 122 A1**
(43) Date of publication of application: **12.07.2017**
(21) Application number: 16150589.6
(22) Date of filing: 08.01.2016
(51) Int. Cl.: C07H 17/08, C07H 1/00

(54) **A NOVEL SYNTHETIC PATHWAY TOWARDS SOLITHROMYCIN AND PURIFICATION THEREOF**

(71) Applicant: LEK Pharmaceuticals d.d., 1526 Ljubljana (SI)
(72) Inventor: The designation of the inventor has not yet been filed
(74) Representative: Kluschanzoff, Harald

(57) **Abstract**

The present invention relates to an efficient route of synthesis of solithromycin and to a method of its purification which obviates the necessity of chromatographic purifications and improves the quality of the product by efficiently removing impurities.

## Description

### Field of the invention

The present invention relates to an efficient route of synthesis of solithromycin and to a method of its purification which obviates the necessity of chromatographic separations and improves the quality of the product by efficiently removing impurities.

### Background of the invention

Macrolide antibacterial agents are characterized by a large lactone ring to which one or more deoxy sugars, usually cladinose and desosamine, are attached. The first generation macrolide, erythromycin, was soon followed by second generation macrolides clarithromycin and azithromycin. Due to widespread of bacterial resistance semi-synthetic derivatives, ketolides, were developed. These, third generation macrolides, to which, for example, belongs telithromycin, are used to treat respiratory tract infections. Currently, a fourth generation macrolide, solithromycin (also known as CEM-101) belonging to the fluoroketolide class is in the pre-registration stage. Solithromycin is more potent than third generation macrolides, is active against macrolide-resistant strains, is well-tolerated and exerts good PK and tissue distribution.

One route of synthesis of solithromycin is disclosed in WO2004/080391 A2. Said route is based on the synthetic strategy disclosed in Tetrahedron Letters, 2005, 46, 1483-1487. It is formally a 10 step linear synthesis starting from clarithromycin. Its characteristics are a late cleavage of cladinose, a hexose deoxy sugar which is in ketolides replaced with a keto group, and a 3-step building up of the side chain. Most intermediates are amorphous or cannot be purified by crystallization, hence chromatographic separations are required.

WO 2009/055557 A1 describes a process, in which the linker part of the side chain (azidobutyl) is synthesized separately thus making the synthesis more convergent. In addition, the benzoyl protection group instead of the acetyl is used to protect the 3-hydroxy group of the tetrahydropyran moiety. The linker part of the side chain is introduced using 4-azidobutanamine which is prepared through a selective Staudinger monoreduction of 1,4-diazidobutane.

WO 2014/145210 A1 discloses several routes of synthesis all based on the use of already fully constructed side chain building blocks, or its protected forms, which are reacted with an imidazoyl carbamate still containing the protected cladinose moiety. After the introduction of the side chain, the cladinose is cleaved and the aniline group protected for the oxidation of the hydroxy group and the fluorination. After fluorination and deprotection or unmasking of the aniline group, solithromycin is obtained.

The object of the present invention is to provide an efficient process for beneficially providing solithromycin and to provide an efficient purification process that would avoid the necessity of using burdensome chromatographic separation techniques.

### Summary of the invention

The object is solved by the methods according to claims 1, 6, 8, 10 and 15, the solithromycin salts according to claims 13 and 14, while preferred embodiments are set forth in dependent claims and will be further described below.

Various aspects, advantageous features and preferred embodiments of the present invention as summarized in the following items, respectively alone or in combination, contribute to solving the object of the invention.
1. A process for the preparation of a compound of formula **3** 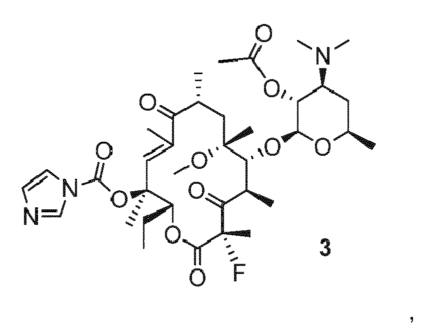 the process comprising the steps of:
   (a) providing a compound of formula 2 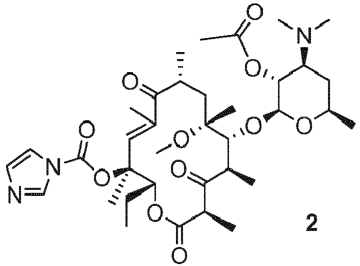 and,
   (b) subjecting the compound of formula **2** to the fluorination reaction to give the compound of formula **3.**
2. The process according to item 1, wherein the fluorination reaction is conducted with an electrophilic fluorination reagent in the presence of a base selected from the group consisting of amidines, alkali salts of *tert*-alcohols, and guanidines.
3. The process according to item 2, wherein the electrophilic fluorination reagent is selected from a group consisting of fluoroimides, fluoroammonium salts and fluoropyridinium salts.
4. The process according to item 3, wherein the electrophilic fluorination reagent is a fluoroimide *N*-fluorobenzenesulfonimide.
5. The process according to item 2, wherein the base is an amidine 1,8-diazabicyclo[5.4.0]undec-7-ene.
6. The process according to item 1, wherein the compound of formula **2** is prepared by a process comprising the steps of:
   (i) providing a compound of formula **1** 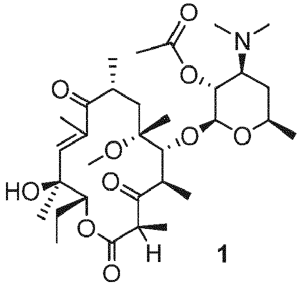 and,
   (ii) reacting the compound of formula 1 with 1,1'-carbonyldiimidazole to give the compound of formula **2.**
7. The process according to item 6, wherein step (ii) is conducted in the presence of 1,8-diazabicyclo[5.4.0]undec-7-ene.
8. A process for the preparation of a compound of formula **3** 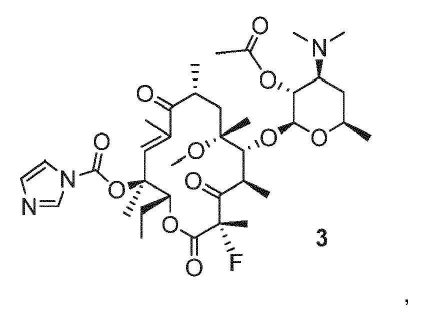 the process comprising the steps of:
   (a') providing a compound of formula **1** 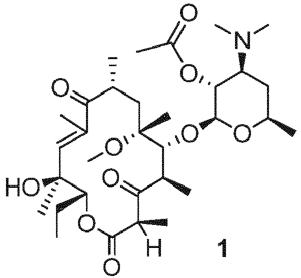
   (b') reacting the compound of formula 1 with 1,1'-carbonyldiimidazole in the presence of 1,8-diazabicyclo[5.4.0]undec-7-ene to give the compound of formula **2** 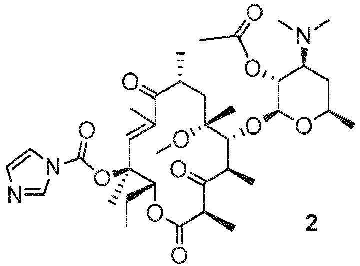 and,
   (c') reacting the compound of formula 2 with *N*-fluorobenzenesulfonimide in the presence of 1,8-diazabicyclo[5.4.0]undec-7-ene to give the compound of formula **3.**
9. The process according to item 8, wherein the compound of formula **2** is not isolated.
10. A process for preparing a compound of formula **5** 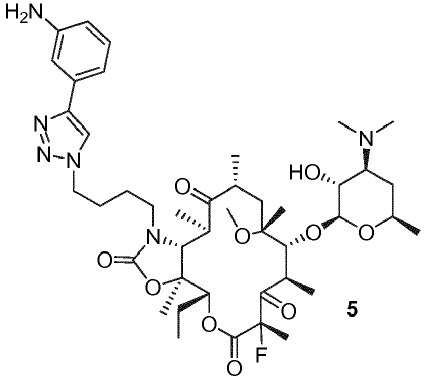 the process comprising the steps of:
   (I) preparing the compound of formula **3** according to any one of previous items,
   (II) reacting the compound of formula **3** with 3-(1-(4-aminobutyl)-1*H*-1,2,3-triazol-4-yl)aniline of formula 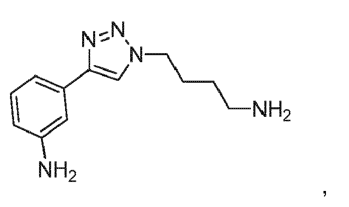 to give the compound of formula **4** 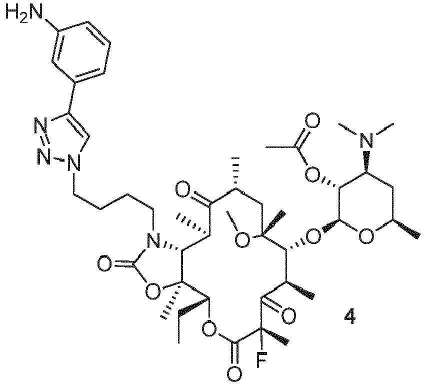 and
   (III) deprotecting the compound of formula **4** to give the compound of formula **5.**
11. The process according to item 9, wherein step (II) is conducted in the presence of MeCN and 1,8-diazabicyclo[5.4.0]undec-7-ene.
12. The process according to item 9, wherein step (III) is conducted in the presence of MeOH.
13. The process according to item 9, wherein the 3-(1-(4-aminobutyl)-1*H*-1,2,3-triazol-4-yl)aniline of formula 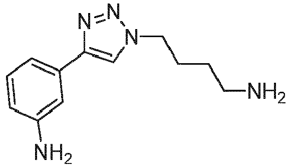 is prepared by a process comprising the steps of:
   o) providing *N*-(4-chlorobutyl)phthalimide of formula 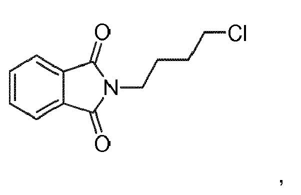
   p) converting the *N*-(4-chlorobutyl)phthalimide to the compound of formula 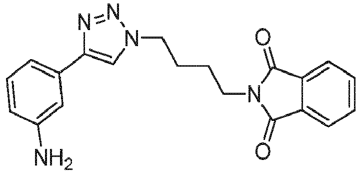 by applying first an azidation step followed by Huisgen cycloaddition step with 3-aminophenylacetylene, and
   r) removing the phthalimido group to give 3-(1-(4-aminobutyl)-1*H*-1,2,3-triazol-4-yl)aniline.
14. The process according to item 13, wherein the azidation step in step p) is performed using sodium azide in DMSO at 40-70 °C.
15. The process according to item 13, wherein in step r) the phthalimido group is removed with hydrazine in n-butanol.
16. A process for purification of the compound of formula **5** 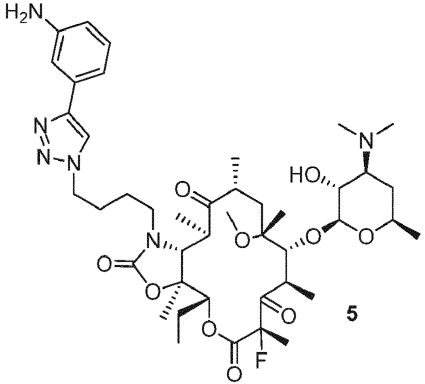 the process comprising the steps of:
   (A) providing the compound of formula **5,**
   (B) converting the compound of formula **5** into its crystalline salt, wherein the crystalline salt is formed with an acid selected from a group consisting of oxalic acid, citric acid, D-(-)-tartaric acid, dibenzoyl-d-tartaric acid, 2,4-dihydroxybenzoic acid, 3,5-dihydroxybenzoic acid, and (R)-(+)-2-pyrrolidinone-5-carboxylic acid,
   (C) dissolving the obtained salt in water,
   (D) washing the obtained aqueous solution of the salt with a solvent selected from a group consisting of dichloromethane, 2-methyltetrahydrofuran, ethyl acetate and 2-butanone,
   (E) basifying the aqueous solution,
   (F) isolating the precipitated pure compound of formula **5,** and
   (G) optionally, crystallizing the obtained pure compound of formula **5** from ethanol.
17. The process according to item 16, wherein in step (A) the compound of formula **5** is provided by a process according to any one of items 1 to 15.
18. The process according to item 16, wherein in step (E) ammonia or sodium carbonate is used for basifying the aqueous solution.
19. The process according to item 16 or item 17, wherein in step (B) the compound of formula **5** is first dissolved in isopropyl acetate or 2-methyltetrahydrofuran, the crystalline salt is formed with oxalic acid, the obtained oxalate salt is in step (D) washed with 2-methyltetrahydrofuran or 2-butanone, and the aqueous solution of the oxalate salt is in step (E) basified with ammonia or sodium carbonate.
20. A crystalline salt of a compound of formula **5** 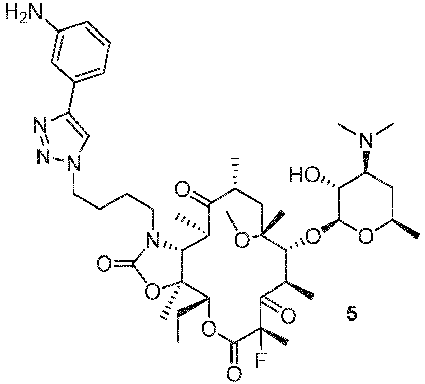 wherein the salt is selected from a group consisting of oxalate, citrate, D-(-)-tartrate, dibenzoyl-d-tartrate, 2,4-dihydroxybenzoate, 3,5-dihydroxybenzoate, and (R)-(+)-2-pyrrolidinone-5-carboxylate.
21. A crystalline solithromycin (a compound of formula **5)** oxalate salt of formula 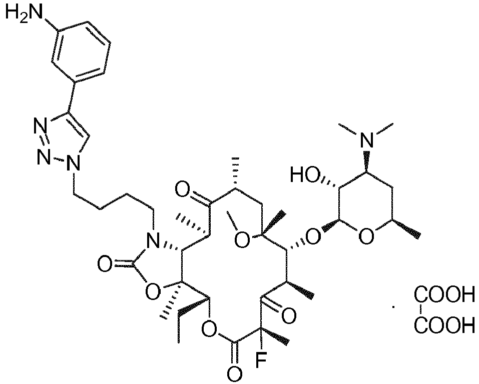
22. A crystalline solithromycin (a compound of formula **5)** citrate salt of formula 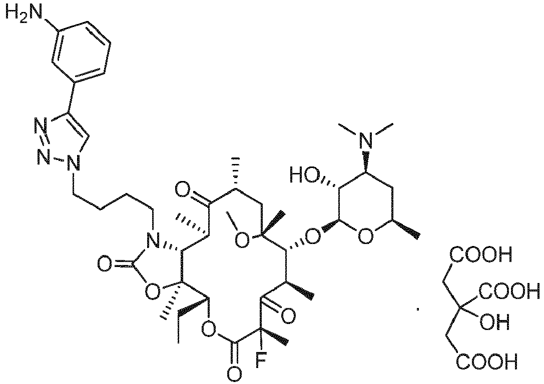
23. A process for the preparation of a pharmaceutical composition comprising a compound of formula **5** 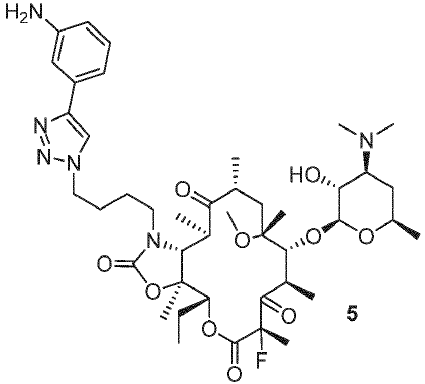 the process comprising the steps of:
   (x) preparing the compound of formula **5** by carrying out the process according to any of items 10 to 19, and
   (y) mixing said obtained compound of formula **5** with a pharmaceutically acceptable carrier and/or excipient.

### Detailed description

In the following, the present invention is described in more detail while referring to preferred embodiments and examples, which are presented however for illustrative purposes and shall not be construed to limit the invention in any way.

The present invention provides an industrially applicable, economical process for the preparation of pure solithromycin. The process employs a fluorination of a carbamate intermediate which improves the process in many aspects, such as enabling a direct introduction of the 4-(4-(3-aminophenyl)triazolyl)butyl side chain while avoiding protecting group chemistry. In addition, the novel fluorination step allows process simplifications, such as merging steps in one-pot or telescoping operations. Moreover, the purification process of the present invention obviates the necessity of chromatographic purifications and improves the quality of the product by efficiently removing impurities. In addition it can be used for recovering solithromycin from the crystallization mother liquors, thus improving yield.

### Macrolide building block synthesis

A first aspect of the invention is a process for providing a macrolide building block, i.e. a compound of formula **3,** as depicted in Scheme 1.

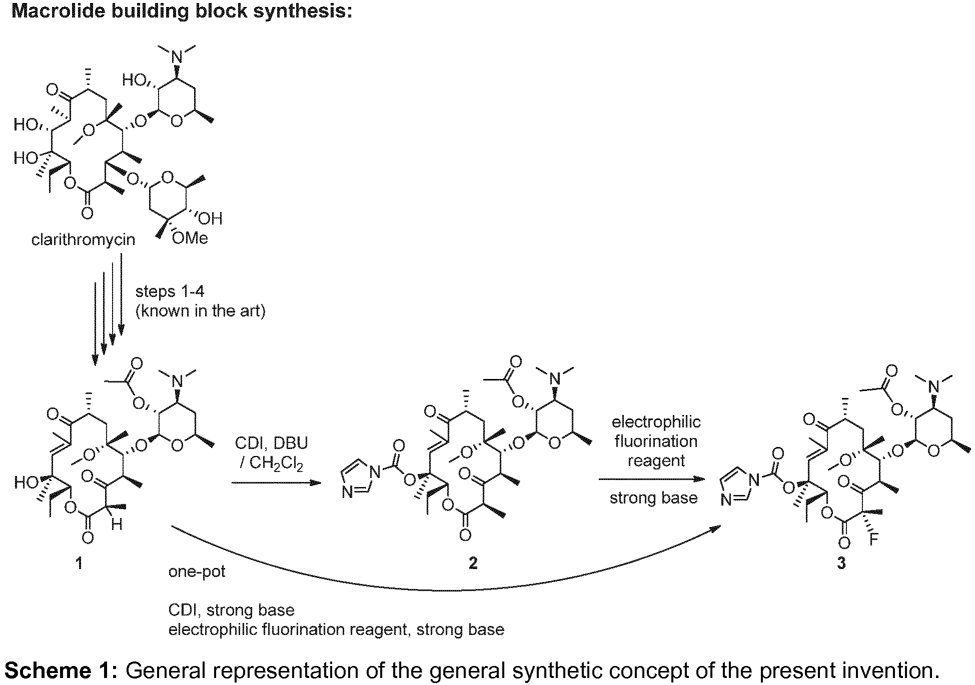

The compound of formula **3** is synthesized by enolate electrophilic fluorination of the carbamate intermediate **2,** which can be prepared from the intermediate **1.** Both the intermediate **1** and **2** can be prepared by known processes, for example, applying transformations from clarithromycin as described, for instance, in WO 2009/053259. In the fluorination reaction, the carbamate macrolide **2** is treated with an electrophilic fluorination reagent in the presence of a strong base required to form the enolate from the 1,3-dicarbonyl structural element. Poorly nucleophilic strong bases are preferred, such as 1,8-diazabicyclo[5.4.0]undec-7-ene (DBU) or related amidines, potassium *tert*-butylate (*^{t}*BuOK) or other alkali salts of *tert*-alcohols, and *2-tert-*butyl-1,1,3,3-tetramethylguanidine or other substituted guanidines. Suitable electrophilic fluorination reagents are *N*-fluorobenzenesulfonimide (NFSI), as well as other fluoroimides, 1-chloromethyl-4-fluoro-1,4-diazoniabicyclo[2.2.2]octane bis(tetrafluoroborate) (Selectfluor^{®}) or other fluoroammonium salts, 2,6-dichloro-1-fluoropyridinium tetrafluoroborate or other fluoropyridinium salts.

The carbamate formation and fluorination can be incorporated in a one-pot procedure where the hydroxy group in **1** is first acylated with 1,1'-carbonyldiimidazole (CDI) in the presence of a strong base, for example, DBU, and then the so formed carbamate **2** is fluorinated with an electrophilic fluorination reagent, for example, NFSI, to give **3** in a one-pot procedure.

Intermediate **2** might be considered as too reactive and fragile to be suitable for the enolate fluorination conditions, because the carbamate is of an azolide type, which means it is reactive toward nucleophiles, some bases, and sensitive under hydrolytic conditions (for example, it slowly hydrolyses in aqueous solutions). Contrary to such expectations, we have now shown that intermediate **2** can be fluorinated without extensive decomposition of the carbamate moiety, which allows for the fluorination to be performed before the incorporation of the side chain. The application of masking or protecting group chemistry to protect the aniline moiety from oxidation caused under fluorinating conditions is thus made unnecessary.

Furthermore, an efficient fluorination of intermediate **2** in the presence of acetyl group in the pyran part of the molecule has not been achieved by the processes known in the art. EP0949268 A1, for example, discloses a process for preparation of intermediate **2,** in which the acetyl protection has to be temporarily exchanged for silyl protection in order the fluorination carried out on the analogue of intermediate **1** is made efficient in view of yields and purity. We have now shown that efficient fluorination of intermediate **2** is possible in the presence of acetyl group in the pyran part of the molecule without exchanging of protection groups. Consequently, the steps of exchanging the protection groups can be omitted, which makes the synthesis of the compound of formula **3** and the compound of formula **5** (solithromycin) even more efficient and industrially applicable.

In a specific embodiment presented in Scheme 2, the carbamate intermediate **(2)** is prepared by using 1,1'-carbonyldiimidazole (CDI) in the presence of DBU. The fluorination is performed with NFSI in the presence of DBU in tetrahydrofuran (THF).

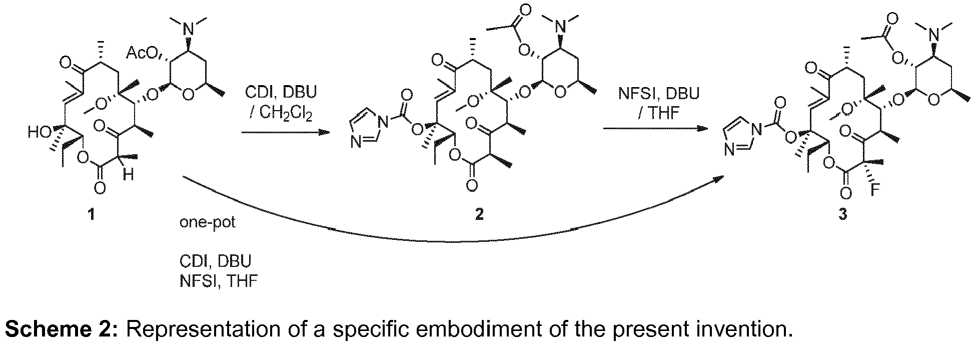

### Side chain building block synthesis

A second aspect of the invention is a process for providing a side chain building block, i.e. 3-(1-(4-aminobutyl)-1*H*-1,2,3-triazol-4-yl)aniline, as depicted in Scheme 3.

Possible synthetic approaches for preparing the side chain are outlined in WO 2014/145210. Specifically, said patent application specifies the route of synthesis for the nitro analogue. A similar route of synthesis was used here for the 3-(1-(4-aminobutyl)-1*H*-1,2,3-triazol-4-yl)aniline, starting, however, from the cheaper 1,4-dichlorobutane instead of the more expensive 1,4-dibromobutane.

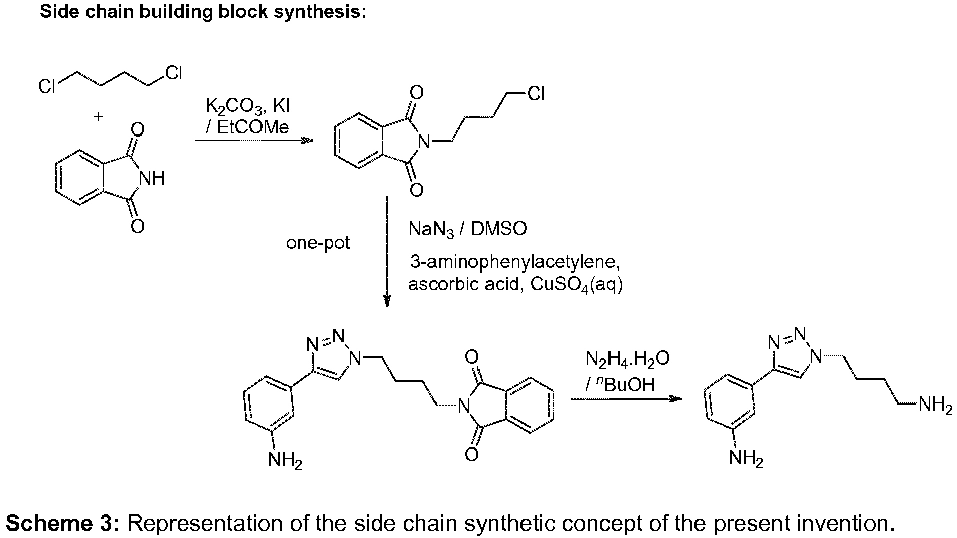

The synthesis starts by monosubstitution on 1,4-dichlorobutane with phthalimide employing nucleophilic catalysis with potassium iodide. Phthalimide in the presence of potassium carbonate or other suitable base can be employed, or the commercially available potassium salt of phthalimide can be used directly with equal efficiency. The most suitable solvent with regard to yields, reaction selectivity, isolation and cost efficiency, was found to be methyl ethyl ketone. This allows for the preparation of *N*-(4-chlorobutyl)phthalimide in 80% yield and a facile recycling of excess 1,4-dichlorobutane when desired. The major impurities present are the disubstitution product 1,4-bis(phthalimid-N-yl)butane and *N*-(4-iodobutyl)phthalimide. The latter (iodo impurity) is considered to be a useful and beneficial substrate for the next step, in which it acts as a source of iodide that is necessary for the nucleophilic catalysis during the azidation. The formation of the disubstitution side product can be suppressed by employing excess of 1,4-dichlorobutane, with the use of 2 equivalents being found as a suitable compromise.

The azidation step is best performed in DMSO at 40-70 °C using sodium azide. The obtained *N*-(4-azidobutyl)phthalimide is preferably not isolated, but further used in the Huisgen cycloaddition at 20-50 °C with 3-aminophenylacetylene and *in situ* formed Cu(I) catalytic species (1-2 mol%) as a one-pot transformation. The reaction product is isolated simply by aqueous dilution and filtration.

The deprotection of the phthalimide group is performed with hydrazine in n-butanol which is then also suitably used as a solvent during the aqueous washes needed to efficiently remove the phthalazine-1,4-dione stoichiometric by-product and other polar impurities while retaining a good yield. Aprotic solvents otherwise typically used for extractions, such as ethyl acetate or dichloromethane, are not as suitable, because the product tends to preferably partition into the aqueous phase. The product, 3-(1-(4-aminobutyl)-1*H*-1,2,3-triazol-4-yl)aniline is crystallized from tetrahydrofuran with the addition of methyl *tert*-butyl ether as the antisolvent.

### Synthesis of crude solithromycin

A third aspect of the invention is a process for providing crude solithromycin (the compound of formula 5) through a convergent synthesis that combines both aforementioned building blocks, the macrolide building block (compound of formula 3) and the side chain building block 3-(1-(4-aminobutyl)-1*H*-1,2,3-triazol-4-yl)aniline prepared as discussed above.

As shown in Scheme 4, first, the compound of formula 3 and 3-(1-(4-aminobutyl)-1*H*-1,2,3-triazol-4-yl)aniline are reacted in the presence of a strong base, for example, DBU, in a suitable solvent, for example, MeCN, to give the compound of formula 4. In the last step, the acetyl protecting group of the hydroxy group located on position *β* to the dimethylamino substituent is cleaved in methanol.

As discussed above, the fluorination is performed in the presence of acetyl group in the pyran part of the molecule and prior to the incorporation of the side chain, which allows that both exchanging the protection group in the pyran part of the molecule and masking or protecting the aniline moiety from oxidation caused under fluorinating conditions can be avoided.

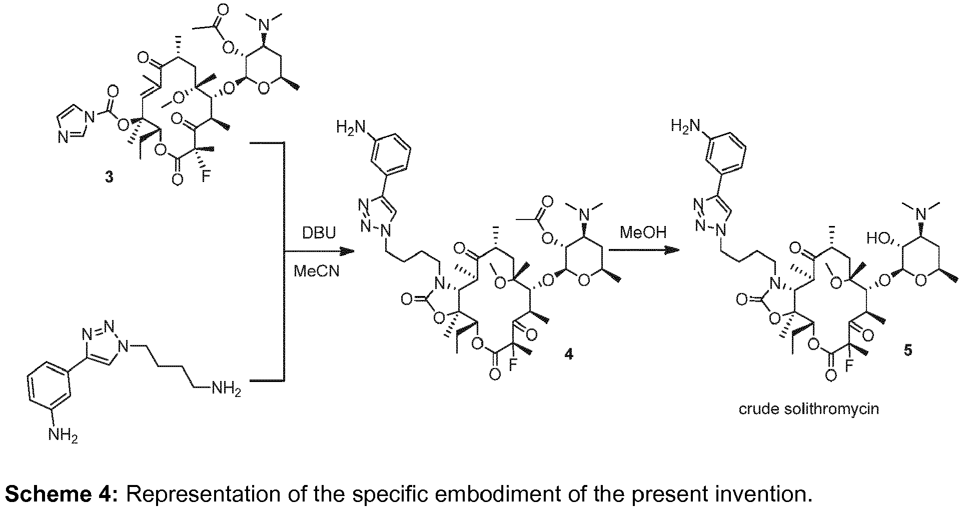

In another aspect of the present invention a process is provided for purification of crude solithromycin.

### Purification of crude solithromycin

Due to its properties, solithromycin is very difficult to purify. The amorphous material obtained after various syntheses is truly a challenge for further processing.

Chromatographic separation is very difficult and of poor resolution. Due to the basic polar functional groups, the compound and its related impurities all tend to "trail" on typical normal stationary phases that are considered suitable for industrial use, such as silica and alumina. Purification by crystallization is just as difficult unless the material is already sufficiently pure. Impurities inhibit its crystallization to such an extent that solutions in alcohols can be stable even in concentrations of several-fold above the saturation levels of the pure material. Such solutions refuse to crystallize even after weeks of stirring or cooling. Seeding with crystalline solithromycin has no effect and the added seeds simply dissolve. In addition, only limited purification is achieved in most solvents. Lower primary alcohols, particularly ethanol, are most efficient for purification by crystallization when this is possible, but give low recovery and the crystallization is most sensitive toward impurities, thus still demanding prior chromatographic separation.

Clearly an alternative method of purification would be advantageous. For this reason we developed a process for purification employing an acidic salt formation, for example solithromycin oxalate salt, freebasing back to solithromycin, and crystallization from ethanol (Scheme 5).

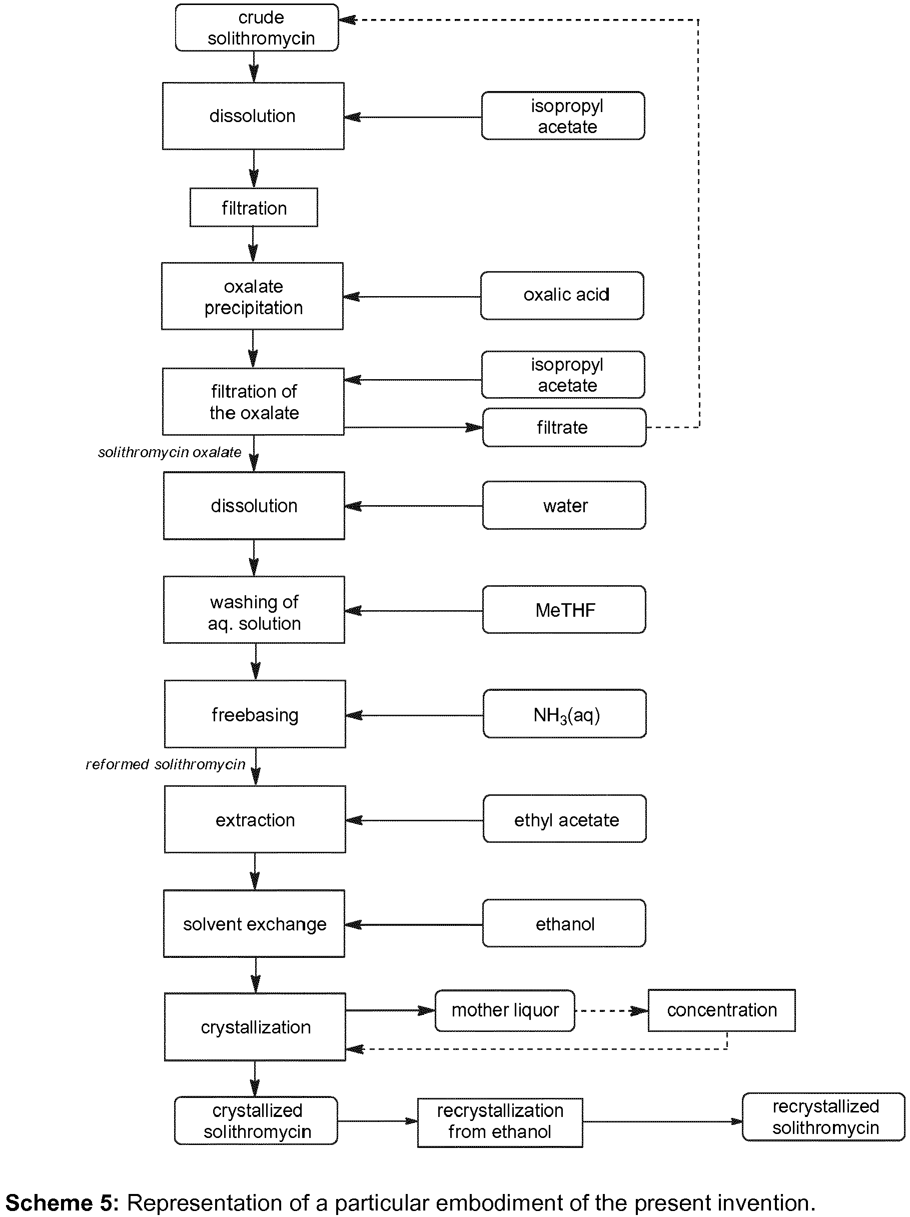

The formation of crystalline salts from crude solithromycin may be inhibited by impurities and is dependent on the solvent used. Only a limited number of acids gave useful precipitates from solutions of crude solithromycin. Of these, the precipitation of the oxalate salt from isopropyl acetate (*ⁱ*PrOAc) or 2-methyltetrahydrofuran (MeTHF) was found to be most efficient in regard to yields, reaction times and purification ability. Precipitation of the citrate salt from MeTHF also significantly increased purity. However, impurities strongly inhibited crystal growth rates, the reaction thus required longer times compared to the oxalate salt formation. Crystallization of salts from solutions of impure solithromycin was also found possible with D-(-)-tartaric, dibenzoyl-*d*-tartaric, 2,4-dihydroxybenzoic, 3,5-dihydroxybenzoic, and (*R*)-(+)-2-pyrrolidinone-5-carboxylic acids using ethyl acetate, isopropyl acetate or MeTHF as solvents, or their mixtures with methyl *t*-butyl ether, but the efficiency and purification abilities were inferior to both the oxalate and the citrate salt.

Some acids, such as (R)-(-)-mandelic, L-(+)-tartaric, *p*-toluenesulfonic, benzoic, malonic, 4-hydroxybenzoic, (-)-malic, and (+)-camphor-10-sulfonic acid formed insoluble amorphous precipitates without improving purity. Many other acids were found unable of forming any precipitate from impure solithromycin under the conditions tested.

The purification *via* the oxalate salt is efficient even in cases of very impure crude solithromycin. Although the selectivity of crystallization of oxalate is not complete as being evident from a number of other impurities that still carry over with the solid precipitate, the product is of such quality that it can be improved by further operations, such as freebasing back to solithromycin and final crystallizations (Table 1). The improvements by such final operations are not successful in a case of crude solithromycin and even in some cases of chromatographic purifications, because the product crystallizes very difficult or even not at all.

The oxalate salt is thus dissolved in water and washed with either MeTHF or 2-butanone, a treatment that efficiently removes impurities less polar than solithromycin. Instead of this washing, a filtration over Celite^{®} (diatomaceous silica) to remove water insoluble resinous materials can be sufficient in some cases. The aqueous solution is then made basic, for example with ammonia or sodium carbonate, and the precipitated solithromycin base is extracted or filtered. Crystallization from ethanol which removes the remaining impurities is thereafter made possible.

**Table 1. Examples of chromatographic purity changes at different stages in various batches.**

| Batch | HPLC chromatographic purity at different purification stages [area%] | | | |
|---|---|---|---|---|
| | Crude solithromycin | Solithromycin oxalate | Reformed solithromycin | Crystallized solithromycin |
| 1 | 28.05 | 49.06 | 55.77 | N/A |
| 2 | 51.19 | 81.47 | 90.03 | 96.40 |
| 3 | 51.19 | 81.93 | N/A | 96.10 |
| 4 | 50.58 | 81.20 | N/A | 87.84 |
| 5 | 70.70 | 81.70 | 87.53 | 96.69 |

The selection of the solvent used for the washing of the aqueous solution of the oxalate salt just before reforming the solithromycin free base is also crucial for improving purity and assuring a crystallisable product. It was found that MeTHF and 2-butanone, two polar but water immiscible solvents, perform best in avoiding emulsion formation and selectivity for impurities removal (Table 2). MeTHF is the preferred solvent. Dichloromethane extracts too much solithromycin, while ethyl acetate is less efficient at removing impurities. Most other solvents were either considerably less efficient or formed emulsions.

**Table 2. An example of how different solvents extract impurities from an aqueous solution of impure solithromycin oxalate.**

| Solvent | Solithromycin chrom. purity [area%] Initial value: 61.58 area% | | Solithromycin partition LogP_{solvent/water} |
|---|---|---|---|
| | Aqueous phase | Solvent phase | |
| Dichloromethane | 72.87 | 48.50 | -0.80 |
| 2-Methyltetrahydrofuran (MeTHF) | 73.72 | 13.46 | -1.20 |
| Ethyl acetate | 69.59 | 17.00 | -1.28 |
| 2-Butanone (MEK) | 72.58 | 23.54 | -0.89 |

Recovery is significantly improved by reintroducing the collected oxalate salt filtrates and crystallization mother liquors back in the process after a proper treatment. Impurities strongly affect the precipitation of the oxalate, thus making the filtrates of the oxalate salt formation step rich in solithromycin. These can be reintroduced at the beginning of the process. The mother liquors from the crystallizations from ethanol can be concentrated and seeded again to give secondary crystals of comparable purity.

**Table 3. Examples of chromatographic purity changes in samples purified by liquid column chromatography (LCC).**

| Batch | HPLC chromatographic purity at different purification stages [area%] | | | |
|---|---|---|---|---|
| | Crude solithromycin | 1^{st} chromatography (LCC, SiO₂) | 2^{nd} chromatography (LCC, RP-18) | Crystallized solithromycin |
| 1 | 74.0^{a} | 91.37 | N/A | 97.96 |
| 2 | 59.50^{b} | 66.72-80.20^{c} | N/A | N/A |
| 3 | 61.70 | N/A | 86.44 | 96.56^{d} |
| 4 | 70.11^{b} | N/A | 86.34 | 94.98 |
| 5 | 60.59 | 88.40 | N/A | 98.51 |
| a) Already purified material by at least one LCC (RP-18 column). | | | | |
| b) Already purified material by at least one LCC (SiO₂ column). | | | | |
| c) Range of purity of fractions collected. N/A - Not applicable. | | | | |
| d) A very small amount of material crystalized out. | | | | |

The results of the oxalate purification process were superior to chromatography based separations, which generally required a separation on silica followed by another separation on reverse phase stationary phase (C-18) in order to obtain a material crystallizable from ethanol (Table 3).

Further aspect of the present invention resides in the provision of valuable crystalline solithromycin salts useful in the purification of solithromycin. Said crystalline salts are formed with an acid selected from a group consisting of oxalic acid, citric acid, D-(-)-tartaric acid, dibenzoyl-*d*-tartaric acid, 2,4-dihydroxybenzoic acid, 3,5-dihydroxybenzoic acid, and (R)-(+)-2-pyrrolidinone-5-carboxylic acid. The preferred crystalline salt of solithromycin is the oxalate salt of formula

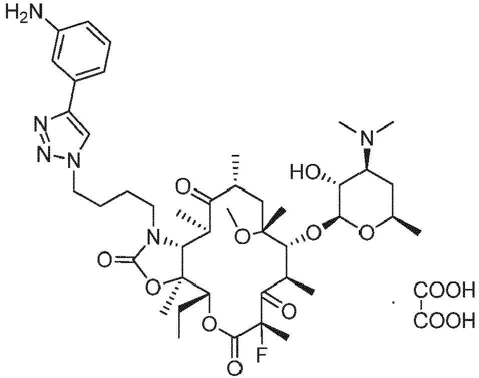

Another aspect of the invention is a process for preparing a pharmaceutical composition comprising solithromycin, the process comprising the steps of carrying out the process of the invention to obtain solithromycin, and mixing solithromycin obtained according to the invention, optionally with another active pharmaceutical ingredient, with a pharmaceutically acceptable excipient, carrier and/or diluent.

In particular, solithromycin is admixed with at least one suitable pharmaceutically acceptable excipient. Pharmaceutically acceptable excipients may be selected from the group consisting of diluents, carriers, binders, disintegrating agents, stabilizing agents, preservatives, lubricants, surfactants, fragrances, flavouring agents, sweeteners and other excipients known in the field of the pharmaceutical technology. For example, suitable excipients may be selected from the group consisting of lactose, microcrystalline cellulose, cellulose derivatives, such as hydroxypropylcellulose, polyacrylates, calcium carbonate, starch, colloidal silicone dioxide, sodium starch glycolate, croscarmellose sodium, talc, magnesium stearate, polyvinylpyrrolidone, polyethylene glycol and other excipients known in the field of the pharmaceutical technology.

Overall, in the present invention a simplified synthesis of solithromycin **(5)** is provided. The fluorination is performed prior to the incorporation of the side chain, which allows that masking or protecting the aniline moiety from oxidation caused under fluorinating conditions can be avoided, thus leading to decreased number of reaction steps. Moreover, efficient fluorination of intermediate **2** is possible even in the presence of acetyl group in the pyran part of the molecule, hence there is no need for exchanging of protection groups. In addition, an advantageous process for purification of crude solithromycin is provided employing an acidic salt formation, preferably solithromycin oxalate salt. Overall, the present invention thus provides cost-effective, environmentally friendly and industrially suitable synthetic process towards pure solithromycin.

### Examples

The following examples are merely illustrative of the present invention and they should not be considered as limiting the scope of the invention in any way. The examples and modifications or other equivalents thereof will become apparent to those versed in the art in the light of the present entire disclosure.

### Example 1: Synthesis of compound 2: (2R,3S,7R,9R,10R,11R,13R,Z)-10-(((2S,3R,4S,6R)-3-acetoxy-4-(dimethylamino)-6-methyltetrahydro-2H-pyran-2-yl)oxy)-2-ethyl-9-methoxy-3,5,7,9,11,13-hexamethyl-6,12,14-trioxooxacyclotetradec-4-en-3-yl 1H-imidazole-1-carboxylate

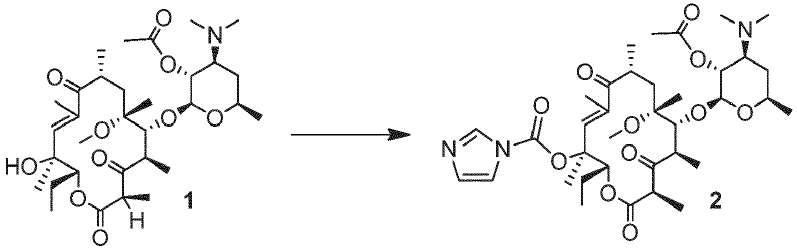

A solution of (2S,3R,4S,6R)-4-(dimethylamino)-2-(((3R,5R,6R,7R,9R,13S,14R,Z)-14-ethyl-13-hydroxy-7-methoxy-3,5,7,9,11,13-hexamethyl-2,4,10-trioxooxacyclotetradec-11-en-6-yl)oxy)-6-methyltetrahydro-2H-pyran-3-yl acetate, **1** (313 g) in dichloromethane (2.22 L) was cooled to-25 °C. DBU (115 mL) followed by CDI (125 g) were added and temperature of the reaction was raised to 0°C. The completion of the reaction was followed by HPLC. Upon the completion, the pH of the reaction mixture was adjusted to 6 using 10% aqueous acetic acid. Layers were separated and organic layer was washed twice with water, dried over sodium sulphate and concentrated to afford compound **2** as white foam (HPLC purity: 90 area%).
¹H NMR (500 MHz, CDCl₃) δ 8.00 (s, 1H), 7.28 (t, *J* = 1.5 Hz, 1H), 6.96 (dd, *J* = 1.6, 0.8 Hz, 1H), 6.70 (s, 1H), 5.58 (dd, *J* = 10.0, 3.2 Hz, 1H), 5.22 (s, 3H), 4.61 (dd, *J* = 10.5, 7.6 Hz, 1H), 4.25 (d, *J* = 7.6 Hz, 1 H), 4.02 (d, *J* = 8.6 Hz, 1 H), 3.67 (q, *J* = 6.8 Hz, 1 H), 3.42-3.37 (m, 1 H), 3.04 (br s, 1 H), 2.93 (quintet, *J* =7.7 Hz, 1 H), 2.67 (s, 3H), 2.58-2.52 (m, 1 H), 2.13 (s, 6H), 1.94 (s, 3H), 1.77 (s, 3H), 1.72 (d, *J* = 0.8 Hz, 3H), 1.64-1.53 (m, 2H), 1.25 (d, *J* = 6.9, 3H), 1.19 (s, 3H), 1.13 (d, *J* = 6.2 Hz, 3H), 1.11 (d, *J* = 6.9 Hz, 3H), 1.02 (d, *J* = 7.4 Hz, 3H), 0.84 (t, *J* = 7.4 Hz, 3H).
¹³C NMR (125 MHz, CDCl₃) δ 204.84, 203.63, 169.64, 168.79, 145.85, 138.48, 137.94, 136.95, 130.75, 117.04, 101.78, 84.45, 80.77, 78.44, 76.89, 71.38, 69.02, 63.37, 50.91, 50.13, 47.31, 40.48, 40.48, 40.16, 38.81, 30.12, 22.53, 21.27, 20.84, 20.56, 20.06, 18.81, 14.98, 13.91, 13.14, 10.37.

### Example 2: Synthesis of compound 3: (2R,3S,7R,9R,10R,11R,13S,Z)-10-(((2S,3R,4S,6R)-3-acetoxy-4-(dimethylamino)-6-methyltetrahydro-2H-pyran-2-yl)oxy)-2-ethyl-13-fluoro-9-methoxy-3,5,7,9,11,13-hexamethyl-6,12,14-trioxooxacyclotetradec-4-en-3-yl 1H-imidazole-1-carboxylate

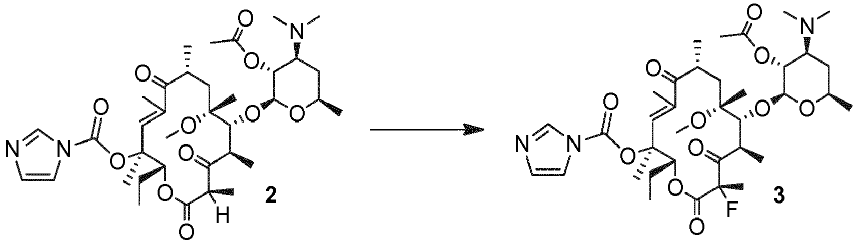

A solution of (2R,3S,7R,9R,10R,11R,13R,Z)-10-(((2S,3R,4S,6R)-3-acetoxy-4-(dimethylamino)-6-methyltetrahydro-2H-pyran-2-yl)oxy)-2-ethyl-9-methoxy-3,5,7,9,11,13-hexamethyl-6,12,14-trioxooxacyclotetradec-4-en-3-yl 1H-imidazole-1-carboxylate, **2** in THF (9 L) was cooled to 0 °C. DBU (115 mL) followed by NFSI (211 g) were added and reaction mixture was stirred at 0°C until completion (followed by HPLC). The reaction mixture was quenched with cold, diluted NaHCO₃ (3 L). DCM (2.5 L) was added and layers were separated. Aqueous layer was washed with additional DCM (1.5 L). Combined organic layers were washed with brine (2 L), dried over sodium sulphate, filtrated and concentrated. Crude material was suspended in *i*PrOAc (2.5 L). Undissolved material was filtered-off and filtrate was concentrated *in vacuo* to afford compound **3** as pale yellow foam (475 g, HPLC purity: 85 area%).
¹⁹F NMR (470 MHz, CDCl₃) δ-163.11.
¹³C NMR (125 MHz, CDCl₃)δ 204.81, 202.27, 169.59, 165.51 (d, *J* = 22.9 Hz), 145.64, 138.02, 137.78, 136.85, 130.69, 116.95, 101.57, 97.86 (d, *J* = 204.5 Hz), 84.23, 80.23, 78.95, 77.97, 71.24, 68.92, 63.11, 49.05, 40.48, 40.48, 40.40, 40.08, 30.22, 24.18 (d, *J* = 16.8 Hz), 22.62, 21.64, 21.18, 20.76, 19.97, 19.39, 14.37, 13.13, 10.32.

### Example 3: Synthesis of compound 3: (2R,3S,7R,9R,10R,11R,13S,Z)-10-(((2S,3R,4S,6R)-3-acetoxy-4-(dimethylamino)-6-methyltetrahydro-2H-pyran-2-yl)oxy)-2-ethyl-13-fluoro-9-methoxy-3,5,7,9,11,13-hexamethyl-6,12,14-trioxooxacyclotetradec-4-en-3-yl 1H-imidazole-1-carboxylate

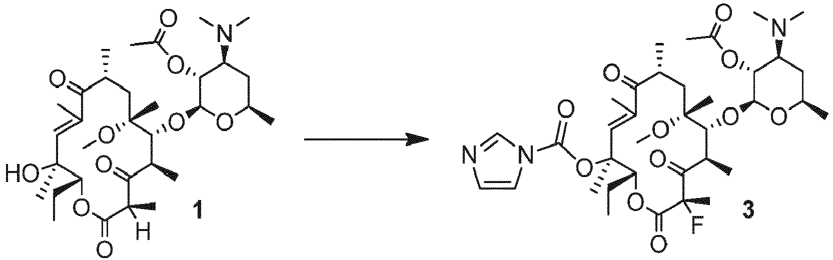

A solution of (2S,3R,4S,6R)-4-(dimethylamino)-2-(((3R,5R,6R,7R,9R,13S,14R,Z)-14-ethyl-13-hydroxy-7-methoxy-3,5,7,9,11,13-hexamethyl-2,4,10-trioxooxacyclotetradec-11-en-6-yl)oxy)-6-methyltetrahydro-2H-pyran-3-yl acetate, **1** (2.45 g) in THF (17 mL) was cooled to 0 °C. DBU (0.9 mL) followed by CDI (0.97 g) were added. The completion of the reaction was followed by HPLC. Upon the completion, reaction was diluted by addition of THF (34 mL). Temperature of the reaction was lowered to -10 °C. DBU (0.72 mL) was added followed by solution of NFSI (1.51 g) in THF (14 mL). Upon completion of the reaction, mixture was diluted with the addition of water/*i*PrOAc (1:4) mixture and layers were separated. Organic phase was washed with water (3 x 25 mL), dried over Na₂SO₄, filtered and concentrated to afford compound **3** as a white foam (3.1 g, HPLC purity: 70 area%).

### Example 4: Synthesis of 3-(1-(4-aminobutyl)-1H-1,2,3-triazol-4-yl)aniline

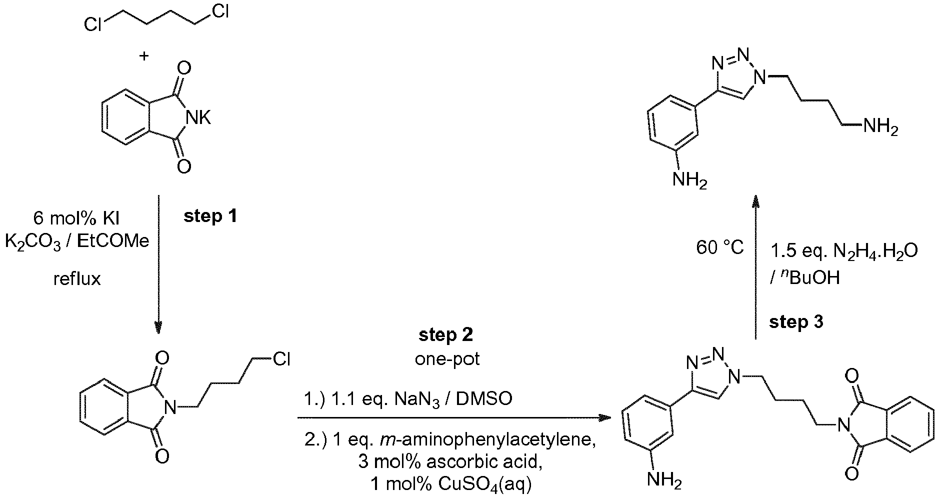

*2-(4-Chlorobutyl)isoindoline-1,3-dione.* A mixture of phthalimide potassium salt (1134 g, 6.00 mol), potassium carbonate (209 g, 1.50 mol), 1,4-dichlorobutane (1555 g, 12.00 mol), and potassium iodide (51 g, 0.30 mol, 5 mol%) in 2-butanone (4.80 L) was stirred 3 days at reflux conditions. The reaction mixture cooled to 40 °C was filtered and the insoluble materials washed with 2-butanone (1.00 L). The filtrate was evaporated at 80 °C under reduced pressure. 2-Propanol (1.00 L) was added to the residue and the solvent removed under reduced pressure. The residue was then crystallized from 2-propanol (4.30 L) at 25 °C. The product was isolated by filtration and washed with 2-propanol (1.00 L). After drying at 40 °C and approximately 50 mbar, there was obtained a white powder (1111 g): 95% assay by quantitative ¹H NMR; MS (ESI) *m*/*z* = 238 [MH]⁺.

*2-(4-(4-(3-Aminophenyl)-1H-1,2,3-triazol-1-yl)butyl)isoindoline-1,3-dione.* To a solution of 2-(4-chlorobutyl)isoindoline-1,3-dione (950 g, 4.00 mol) in DMSO (2.80 L) was added sodium azide (305 g) and the mixture stirred 4 h at 70 °C. The reaction temperature was reduced to 25 °C and there was added in this order water (0.80 L), ascorbic acid (43 g, 0.24 mol, 6 mol%), 0.5M CuSO₄(aq) (160 mL, 2 mol%) and m-aminophenylacetylene (493 g, 4.00 mol). The resulting mixture was stirred 18 h at 40 °C, forming a thick yellow slurry, which was then cooled to 0 °C and slowly diluted with water (2.40 L). The product was isolated by filtration, washing the filter cake with water (3 × 2.00 L) and a 1 : 1 (vol.) mixture of methanol and water (2.00 L). After drying at 50 °C and approximately 50 mbar the product was obtained as a yellow powder (1405 g): 85% assay by quantitative ¹H NMR; MS (ESI) *m*/*z* = 362 [MH]⁺.

*3-(1-(4-Aminobutyl)-1H-1,2,3-triazol-4-yl)aniline.* To a stirred suspension of 2-(4-(4-(3-Aminophenyl)-1*H*-1,2,3-triazol-1-yl)butyl)isoindoline-1,3-dione (659 g, 1.55 mol) in 1-butanol (3.26 L) was added hydrazine hydrate (50-60%, 174 mL). After stirring for 18 h at 60 °C there was added toluene (0.72 L) and 1 M NaOH(aq) (5.00 L). After stirring for 20 min at 60 °C, the aqueous phase was removed and the organic phase washed at this same temperature with 1 M NaOH(aq) (1.00 L), saturated NaCl(aq) (2 × 2.00 L), and concentrated under reduced pressure to 2/3 of the initial quantity, dried over anhydrous sodium sulfate (300 g) in the presence of Fluorisil (30 g), filtered and evaporated under reduced pressure at 70 °C. The residual 1-butanol is removed azotropically by adding toluene and evaporation under reduced pressure (2 × 0.50 L). The residue was dissolved in tetrahydrofuran (0.50 L). To this solution kept stirring at 25 °C was slowly added methyl t-butyl ether (0.50 L) at which point the mixture was seeded. Additional methyl t-butyl ether (0.50 L) was slowly added and the product isolated by filtration, washed with methyl t-butyl ether (0.50 L), and dried at 35 °C and approximately 50 mbar to give an amber colored powder (321 g): mp = 70-73 °C (DSC);
¹H NMR (DMSO-d₆, 500 MHz) 1.30 (m, 2H), 1.86 (m, 2H), 2.3-2.1 (bs, 2H), 2.53 (t, *J* = 6.0 Hz, 2H), 4.35 (t, *J* = 7.1 Hz, 2H), 5.17 (bs, 2H), 6.51 (ddd, *J* = 8.0, 2.3, 1.0 Hz, 1 H), 6.92 (m, 1 H), 7.05 (t, *J* = 7.8 Hz, 1H), 7.09 (t, *J* = 1.9 Hz, 1H), 8.39 (s, 1H); 98% assay by quantitative ¹H NMR; MS (ESI) *m*/*z* = 232 [MH]⁺; IR (NaCl) 694, 789, 863, 1220, 1315, 1467, 1487, 1590, 2935 cm⁻¹.

### Example 5: Synthesis of compound 4: (2S,3R,4S,6R)-2-(((3aS,4R,7S,9R,10R,11 R,13R, 15R)-1-(4-(4-(3-aminophenyl)-1 H-1,2,3-triazol-1-yl)butyl)-4-ethyl-7-fluoro-1-methoxy-3a,7,9,11,13,15-hexamethyl-2,6,8,14-tetraoxotetradecahydro-1 H-[1]oxacyclo-tetradecino[4,3-d]oxazol-10-yl)oxy)-4-(dimethylamino)-6-methyltetrahydro-2H-pyran-3-yl acetate

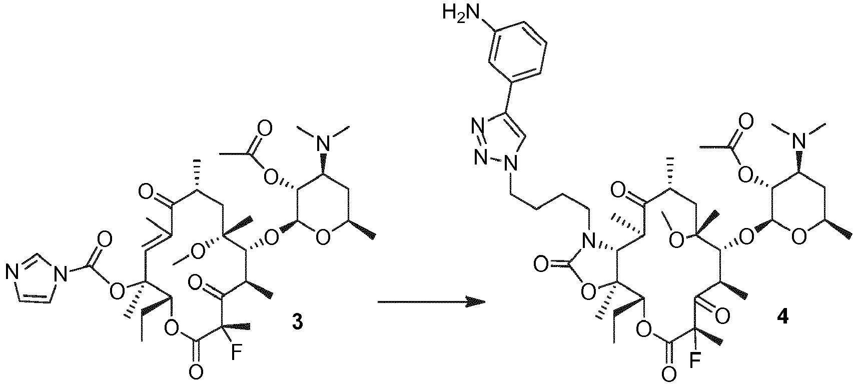

A solution of (2R,3S,7R,9R,10R,11R,13S,Z)-10-(((2S,3R,4S,6R)-3-acetoxy-4-(dimethylamino)-6-methyltetrahydro-2H-pyran-2-yl)oxy)-2-ethyl-13-fluoro-9-methoxy-3,5,7,9,11,13-hexamethyl-6,12,14-trioxooxacyclotetradec-4-en-3-yl 1H-imidazole-1-carboxylate (425 g) in acetonitrile (3.1 L) was cooled to 0 °C. 4-(1-(4-aminobutyl)-1H-1,2,3-triazol-4-yl)aniline, **3** (213 g) followed by DBU (83 mL) were added. The mixture was stirred at 0° C until completion of the reaction (followed by HPLC). Mixture of DCM and water was added (4 L, 1:1) and the pH was adjusted to 6 with 10% aqueous acetic acid. Layers were separated and organic layer was washed with water (2 x 2 L), dried over sodium sulphate and concentrated. The crude material was suspended in EtOAc (2.5 L). Undissolved material was filtered off and filtrate was concentrated *in vacuo* to afford compound **4** as yellow foam (470 g, HPLC purity: 75 area%).
¹⁹F NMR (470 MHz, CDCl₃) δ -164.17 (q, *J* = 21.3 Hz).
¹³C NMR (125MHz, CDCl₃) δ 216.67, 202.47 (d, *J* = 28.2 Hz), 169.88, 166.44 (d, *J* = 23.1 Hz), 157.28, 147.92, 147.00, 131.81, 129.73, 119.80, 116.16, 114.81, 112.42, 101.90, 98.02 (d, *J* =205.9 Hz), 82.18, 79.74, 78.72, 71.68, 69.33, 63.33, 61.13, 49.80, 49.31, 44.61, 42.85, 40.71, 39.37, 39.28, 31.97, 30.53, 29.11, 27.69, 25.24 (d, *J* = 22.2 Hz), 24.36, 22.78, 22.24, 21.49, 21.04, 19.80, 18.04, 14.78, 14.70,14.21, 13.83, 10.57.

### Example 6: Synthesis of compound 5 (solithromycin): (3aS,4R,7S,9R,10R,11R,13R,15R)-1-(4-(4-(3-aminophenyl)-1H-1,2,3-triazol-1-yl)butyl)-10-(((2S,3R,4S,6R)-4-(dimethylamino)-3-hydroxy-6-methyltetrahydro-2H-pyran-2-yl)oxy)-4-ethyl-7-fluoro-11-methoxy-3a,7,9,11,13,15-hexamethyloctahydro-1 H-[1]oxacyclotetradecino[4,3-d]oxazole-2,6,8,14(7H,9H)-tetraone

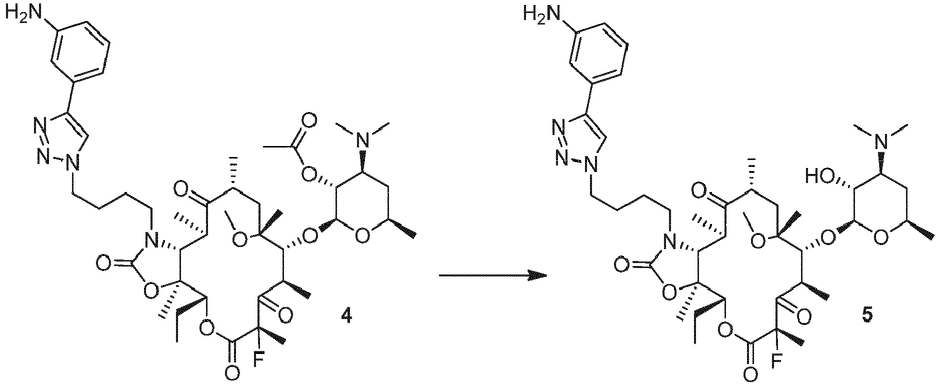

A solution of (2S,3R,4S,6R)-2-(((3aS,4R,7S,9R,10R,11R,13R,15R)-1-(4-(4-(3-aminophenyl)-1H-1,2,3-triazol-1-yl)butyl)-4-ethyl-7-fluoro-11-methoxy-3a,7,9,11,13,15-hexamethyl-2,6,8,14-tetraoxotetradecahydro-1H-[1]oxacyclotetradecino[4,3-d]oxazol-10-yl)oxy)-4-(dimethylamino)-6-methyltetrahydro-2H-pyran-3-yl acetate, **4** (470 g) in methanol (4.7 L) was stirred at room temperature and completion of the reaction was followed by HPLC. Upon completion, the reaction mixture was concentrated to afford crude solithromycin 5 as orange foam (402 g, HPLC purity: 73 area%).

### Example 7: Purification of compound 5 (solithromycin): (3aS,4R,7S,9R,10R,11R,13R,15R)-1-(4-(4-(3-aminophenyl)-1 H-1,2,3-triazol-1-yl)butyl)-10-(((2S,3R,4S,6R)-4-(dimethylamino)-3-hydroxy-6-methyltetrahydro-2H-pyran-2-yl)oxy)-4-ethyl-7-fluoro-11-methoxy-3a,7,9,11,13,15-hexamethyloctahydro-1 H-[1]oxacyclotetradecino[4,3-d]oxazole-2,6,8,14(7H,9H)-tetraone

Crude solithromycin 5 (106 g, HPLC purity: 71%) was suspended in *i*PrOAc (3 L) and heated to reflux, then filtered to remove undissolved material. Oxalic acid (8 g) was added to a filtrate at 60°C. Mixture was slowly cooled to RT and left stirring for additional 1 h. Precipitate was filtered off and dried *in vacuo.* Oxalate salt was suspended in water (1.3 L) (if needed mixture was first filtered through Celite) then 25% aqueous ammonia (21 mL) was added and mixture was stirred additional 15 minutes. Precipitate was filtered off, rinsing with water. Wet solithromycin was dissolved it EtOAc (1.8 L)/water (800 mL) solution. Layers were separated and organic phase was washed with water (2 x 250 mL), dried over Na₂SO₄ and filtered. EtOAc was removed *in vacuo* to afford yellow foam.

Yellow foam was dissolved in EtOH (230 mL) and left stirring at RT until white precipitate fell out. White precipitate was dried *in vacuo* at room temperature to afford clean material (HPLC purity 97 area%).
¹⁹F NMR (470 MHz, CDCl₃) δ -164.25 (q, *J* = 21.3 Hz).
¹³C NMR (125 MHz, CDCl₃) δ 216.64, 202.90 (d, *J* = 28.2 Hz), 166.53 (d, *J* = 23.2 Hz), 157.27, 147.88, 146.99, 131.76, 129.70, 119.79, 116.11, 114.79, 112.38, 104.31, 97.84 (d, *J* = 206.1 Hz), 82.19, 80.77, 78.65, 78.58, 70.41, 69.71, 65.84, 61.09, 49.77, 49.28, 44.64, 42.84, 40.92, 40.30, 39.61, 39.26, 28.17, 27.66, 25.29 (d, *J* = 22.5 Hz), 24.32, 22.20, 21.23, 19.82, 17.96, 15.12, 14.77, 13.83, 10.54.

### Example 8: Oxalate salt from crude solithromycin (compound 5)

Crude solithromycin (3.38 g, 51.19 area%) was dissolved in MeTHF (100 mL), the solution filtered, and stirred at 25 °C. To this oxalic acid (182 mg, 2 mmol) was added in one batch. The so formed suspension was stirred for 30 min and the precipitate was isolated by filtration and dried at 40 °C under reduced pressure to obtain a yellowish powder (1.81 g): 81.21 area% by HPLC (UV at 228 nm).

### Example 9: Citrate salt from crude solithromycin (compound 5)

Crude solithromycin (1.17 g, 72.26 area%) was dissolved in MeTHF (30 mL), the solution filtered, and stirred at 25 °C. To this citric acid (193 mg, 1 mmol) was added in one batch. The so formed solution was stirred for 2 days. The formed precipitate was isolated by filtration and dried at 40 °C under reduced pressure to obtain a yellowish powder (0.89 g): 83.39 area% by HPLC (UV at 228 nm).

### Example 10: Purification of crude solithromycin (compound 5) via oxalate (1)

To isopropyl acetate (4.5 L) crude solithromycin (151 g, 51.19 area%) was added, afterwards the mixture was stirred at 80 °C and filtered to remove any insoluble material. The filtrate was then stirred at 50 °C and oxalic acid (11.4 g, 125 mmol) was added in one batch. In less than 1 min a precipitate started to form. The suspension was cooled to 20 °C in the course of 1 h and the product was isolated by filtration, followed by washing with isopropyl acetate (0.5 L), and drying at 40 °C under reduced pressure. There was obtained the oxalate salt as a yellowish powder (94 g): 81.47 area% by HPLC (UV at 228 nm). The evaporation of the filtrate gave a resinous material containing solithromycin that can be recovered by reprocessing (45 g, 51.49 area%).

The above oxalate salt (94 g) was dissolved in water (1.95 L) and filtered over celite. Aqueous ammonia (25%; 30 mL) was then added to the filtrate stirred at 25 °C. After 15 min, the precipitate was filtered and washed with water (0.5 L). The wet filter cake was dissolved in a mixture of ethyl acetate (2.7 L) and water (1.2 L) by stirring for 1.5 h at 25 °C. The organic phase is separated and the aqueous phase was extracted with an additional amount of ethyl acetate (0.4 L). The combined organic phases were dried over Na₂SO₄ and evaporated under reduced pressure. To the residue ethanol (0.2 L) was added and again evaporated to affect the solvent exchange. The residue (57 g) was then dissolved in ethanol (300 mL) and the solution was seeded with crystals of solithromycin (20 mg). After stirring for 18 h at 25 °C, the crystallized product was isolated by filtration and drying at 40 °C under reduced pressure to give solithromycin as an off-white crystalline solid (34 g): 96.40 area% by HPLC (UV at 228 nm).

### Example 11: Purification of crude solithromycin (compound 5) via oxalate (2)

To isopropyl acetate (5.77 L) crude solithromycin (192 g, 72 area%) was added, afterwards the mixture was stirred at reflux and filtered to remove any insoluble material. The filtrate was then stirred at 55 °C and oxalic acid (14.91 g, 164 mmol) was added in one batch. The suspension was cooled to 20 °C in the course of 1 h, stirred for additional 1 h and the product was isolated by filtration, washed with isopropyl acetate (0.5 L), and dried at 40 °C under reduced pressure to give the oxalate salt (106 g): 87.81 area% by HPLC (UV at 228 nm). The evaporation of the filtrate gave a resinous material containing solithromycin that can be recovered by reprocessing (88 g, 61.13 area%).

The above oxalate salt (106 g) was dissolved in water (2.40 L) and washed with MeTHF (2 × 1.00 L) and ethyl acetate (0.50 L). Aqueous ammonia (25%; 37 mL) was then added to the filtrate while stirring at 25 °C. The precipitated product was extracted with ethyl acetate two times (3.00 L and 0.50 L). The combined extracts were washed with water (0.50 L), dried over Na₂SO₄ and evaporated under reduced pressure. To the residue ethanol (2 × 0.4 L) was added and again evaporated to affect the solvent exchange. The residue was then dissolved in ethanol (300 mL). After stirring for 24 h at 25 °C, the crystallized product was isolated by filtration and drying at 40 °C under reduced pressure to give solithromycin as an off-white crystalline solid (42 g): 98.61 area% by HPLC (UV at 228 nm).

The filtrate was evaporated under reduced pressure to give a yellowish foam (29 g: 68.65 area% by HPLC (UV at 228 nm) that was used for reprocessing.

### Example 12: Solithromycin oxalate salt preparation

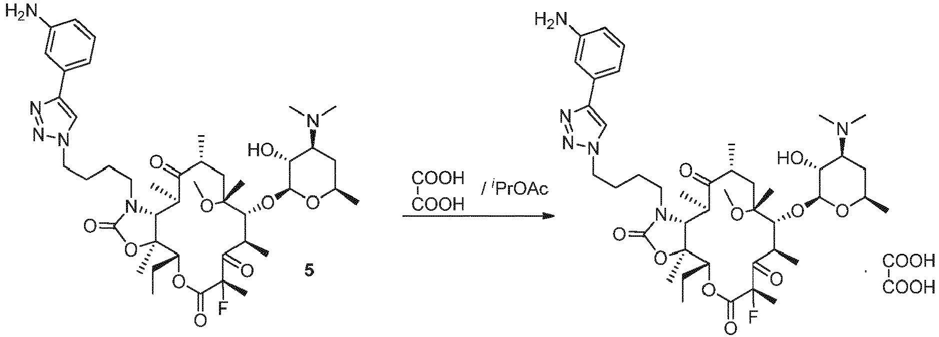

Solithromycin (compound **5)** (719 mg, 0.80 mmol; 93.96 area%) was dissolved in isopropyl acetate (40 mL) by applying heat. The solution was cooled to 40 °C and slowly added during 5 min to a solution of oxalic acid (76 mg, 0.84 mmol) in isopropyl acetate (20 mL) stirring at room temperature. The so formed white suspension was stirred for additional 30 min. The product was then isolated by filtration, washed with isopropyl acetate (15 mL) and dried for 18 h at 40 °C at approximately 50 mbar to give a white crystalline powder (710 mg, 95%): mp 129-135 °C (DSC); 95.07 area% by HPLC (UV at 228 nm); 93% assay by quantitative NMR (the 1 : 1 ratio of solithromycin vs. oxalic acid was confirmed by quantitative ¹³C NMR with long relaxation delays); 1-2 µm particle size under microscope; soluble in water, methanol and DMSO.

The filtrate was evaporated under reduced pressure to give a white solid residue (50 mg): 90.26 area% by HPLC.

### Example 13: Solithromycin D-(-)-tartrate salt preparation

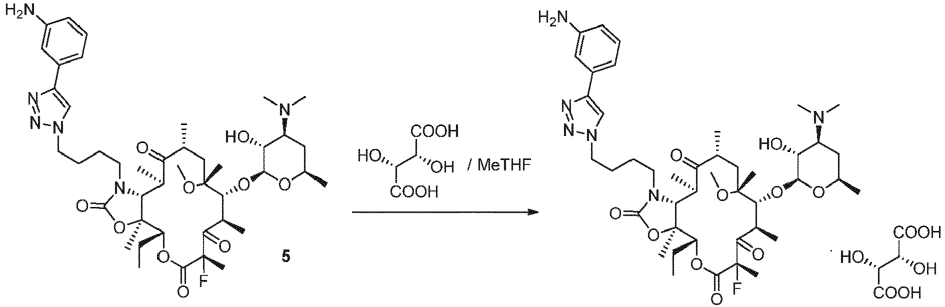

Solithromycin (compound **5)** (719 mg, 0.80 mmol; 93.96 area%) was dissolved in 2-methyltetrahydrofuran (20 mL) by applying heat. The solution was cooled to 40 °C and slowly added during 5 min to a solution of D-(-)-tartaric acid (127 mg, 0.84 mmol) in 2-methyltetrahydrofuran (20 mL) stirring at room temperature. The so formed white suspension was stirred for additional 30 min. The product was then isolated by filtration, washed with 2-methyltetrahydrofuran (10 mL) and dried for 22 h at 40 °C at approximately 50 mbar to give a white crystalline powder (680 mg, 85%): mp = 144-148 °C; 94.17 area% by HPLC (UV at 228 nm); 98% assay by quantitative NMR (the 1 : 1 ratio of solithromycin vs. tartaric acid is confirmed by ¹H NMR); 1-2 µm particle size under microscope; soluble in water, methanol and DMSO.

The filtrate was evaporated under reduced pressure to give a white solid residue (170 mg): 91.97 area% by HPLC (UV at 228 nm).

### Example 14. Solithromycin citrate salt preparation

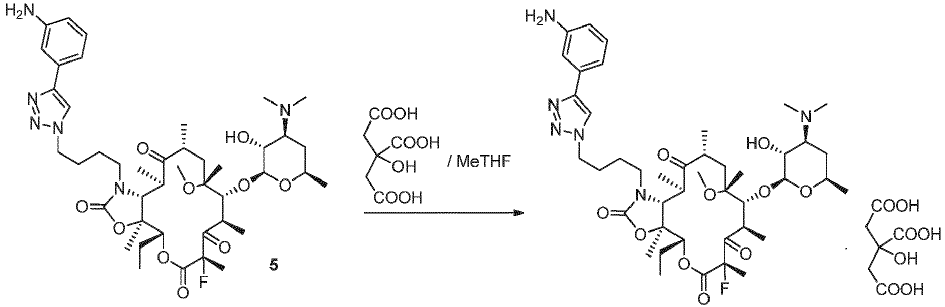

Solithromycin (225 mg, 0.25 mmol; 93.96 area%) was dissolved in 2-methyltetrahydrofuran (4 mL) by applying heat. The solution was cooled to 40 °C and slowly added during 5 min to a solution of citric acid (51 mg, 0.26 mmol) in 2-methyltetrahydrofuran (4 mL) stirring at room temperature. The so formed white suspension was stirred for additional 30 min. The product was then isolated by filtration, washed with 2-methyltetrahydrofuran (4 mL) and dried for 18 h at 40 °C at approximately 50 mbar to give a white crystalline powder (242 mg, 93%): mp = 149-153 °C; 94.14 area% by HPLC (UV at 228 nm); 98% assay by quantitative NMR (the 1 : 1 ratio of solithromycin vs. citric acid was confirmed by quantitative ¹³C NMR with long relaxation delays); 1-3 µm particle size under microscope; soluble in water, methanol, ethanol, acetone and DMSO.

The filtrate was evaporated under reduced pressure to give a resinous residue (38 mg): 90.33 area% by HPLC (UV at 228 nm).

## Claims

1. A process for the preparation of a compound of formula **3** 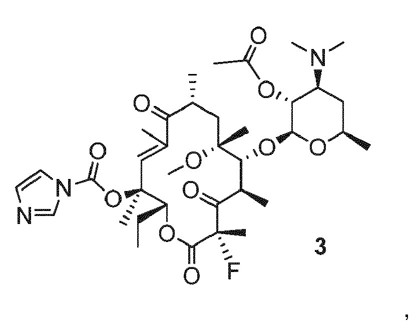 the process comprising the steps of:
(a) providing a compound of formula **2** 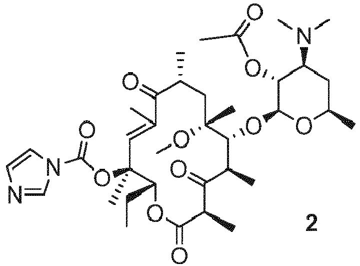 and
(b) subjecting the compound of formula **2** to the fluorination reaction to give the compound of formula **3.**

2. The process according to claim 1, wherein the fluorination reaction is conducted with an electrophilic fluorination reagent in the presence of a base selected from the group consisting of amidines, alkali salts of *tert*-alcohols, and guanidines.

3. The process according to claim 2, wherein the electrophilic fluorination reagent is N-fluorobenzenesulfonimide and the base is 1,8-diazabicyclo[5.4.0]undec-7-ene.

4. The process according to claim 1, wherein the compound of formula **2** is prepared by a process comprising the steps of:
(i) providing a compound of formula **1** 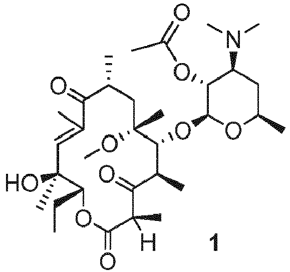 and
(ii) reacting the compound of formula **1** with 1,1'-carbonyldiimidazole to give the compound of formula **2.**

5. The process according to claim 4, wherein step (ii) is conducted in the presence of 1,8-diazabicyclo[5.4.0]undec-7-ene.

6. A process for the preparation of a compound of formula **3** 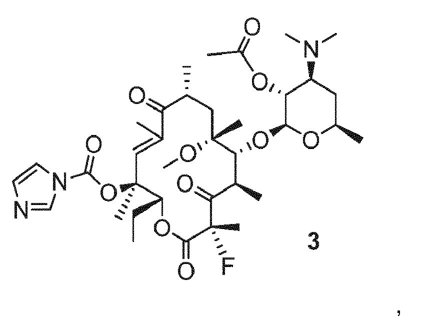 the process comprising the steps of:
(a') providing a compound of formula **1** 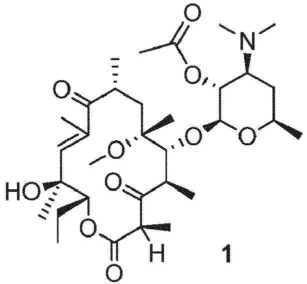
(b') reacting the compound of formula 1 with 1,1'-carbonyldiimidazole in the presence of 1,8-diazabicyclo[5.4.0]undec-7-ene to give the compound of formula **2** 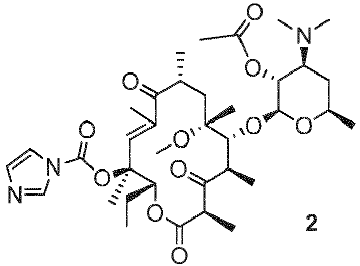 and
(c') reacting the compound of formula **2** with *N*-fluorobenzenesulfonimide in the presence of 1,8-diazabicyclo[5.4.0]undec-7-ene to give the compound of formula **3.**

7. The process according to claim 6, wherein the compound of formula **2** is not isolated.

8. A process for preparing a compound of formula **5** 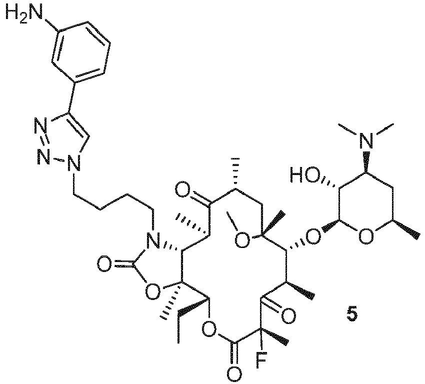 the process comprising the steps of:
(I) preparing the compound of formula **3** according to any one of previous claims,
(II) reacting the compound of formula **3** with 3-(1-(4-aminobutyl)-1*H*-1,2,3-triazol-4-yl)aniline of formula 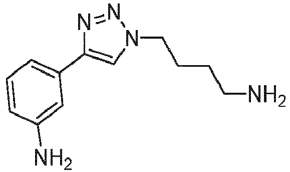 to give the compound of formula **4** 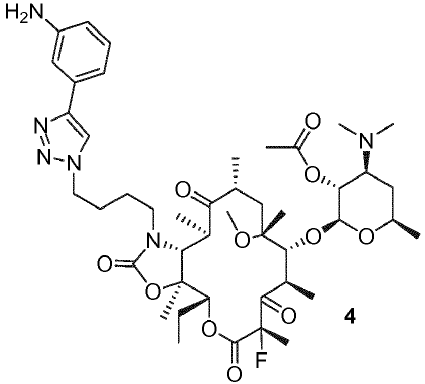 and
(III) deprotecting the compound of formula **4** to give the compound of formula **5.**

9. The process according to claim 8, wherein step (II) is conducted in the presence of MeCN and 1,8-diazabicyclo[5.4.0]undec-7-ene, and step (III) is conducted in the presence of MeOH.

10. A process for purification of the compound of formula **5** 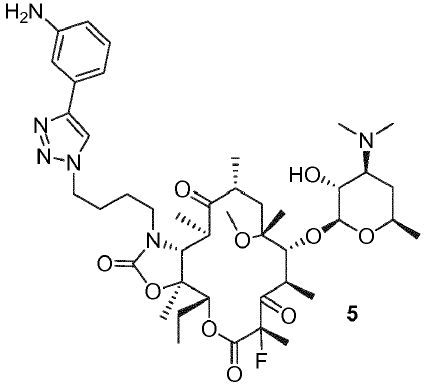 the process comprising the steps of:
(A) providing the compound of formula **5,**
(B) converting the compound of formula **5** into its crystalline salt, wherein the crystalline salt is formed with an acid selected from a group consisting of oxalic acid, citric acid, D-(-)-tartaric acid, dibenzoyl-d-tartaric acid, 2,4-dihydroxybenzoic acid, 3,5-dihydroxybenzoic acid, and (*R*)-(+)-2-pyrrolidinone-5-carboxylic acid,
(C) dissolving the obtained salt in water,
(D) washing the obtained aqueous solution of the salt with a solvent selected from a group consisting of dichloromethane, 2-methyltetrahydrofuran, ethyl acetate and 2-butanone,
(E) basifying the aqueous solution,
(F) isolating the precipitated pure compound of formula **5,** and
(G) optionally, crystallizing the obtained pure compound of formula **5** from ethanol.

11. The process according to claim 10, wherein in step (A) the compound of formula **5** is provided by a process according to any one of claims 1 to 9.

12. The process according to claim 10 or claim 11, wherein in step (B) the compound of formula **5** is first dissolved in isopropyl acetate or 2-methyltetrahydrofuran, the crystalline salt is formed with oxalic acid, the obtained oxalate salt is in step (D) washed with 2-methyltetrahydrofuran or 2-butanone, and the aqueous solution of the oxalate salt is in step (E) basified with ammonia or sodium carbonate.

13. A crystalline salt of a compound of formula **5** 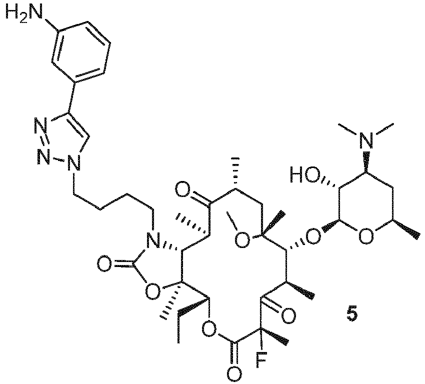 wherein the salt is selected from a group consisting of oxalate, citrate, D-(-)-tartrate, dibenzoyl-*d*-tartrate, 2,4-dihydroxybenzoate, 3,5-dihydroxybenzoate, and (R)-(+)-2-pyrrolidinone-5-carboxylate.

14. A crystalline oxalate salt of formula

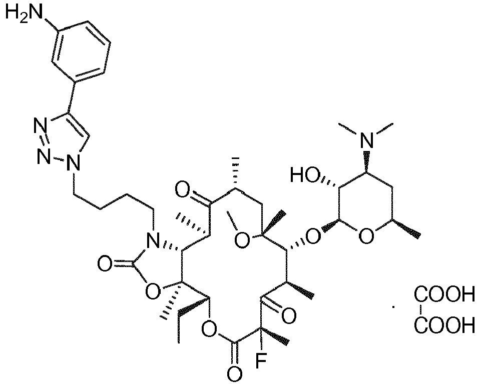

15. A process for the preparation of a pharmaceutical composition comprising a compound of formula **5** 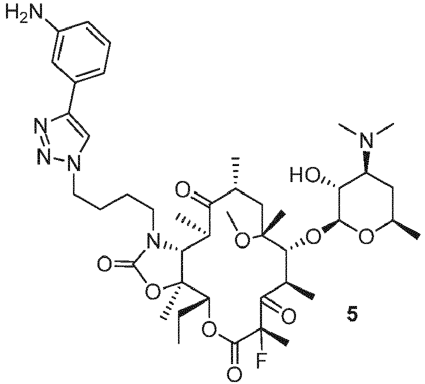 the process comprising the steps of:
(x) preparing the compound of formula **5** by carrying out the process according to any of claims 8 to 12, and
(y) mixing said obtained compound of formula **5** with a pharmaceutically acceptable carrier and/or excipient.
